(19) **Europäisches Patentamt / European Patent Office / Office européen des brevets**

(11) **EP 4 631 950 A1**

(12) **EUROPEAN PATENT APPLICATION**
published in accordance with Art. 153(4) EPC

(43) Date of publication:
**15.10.2025 Bulletin 2025/42**

(21) Application number: **23900655.4**

(22) Date of filing: **05.12.2023**

(51) International Patent Classification (IPC):
*C07H 13/06* (2006.01)   *A23C 13/14* (2006.01)
*A23D 7/00* (2006.01)    *A23F 5/00* (2025.01)
*A23G 3/34* (2006.01)    *A23L 9/20* (2016.01)

(52) Cooperative Patent Classification (CPC):
**A23C 13/14; A23D 7/00; A23F 5/00; A23G 3/34;**
**A23L 9/20; C07H 13/06**

(86) International application number:
**PCT/JP2023/043468**

(87) International publication number:
**WO 2024/122540 (13.06.2024 Gazette 2024/24)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **06.12.2022 JP 2022194655**
**16.06.2023 JP 2023099241**

(71) Applicant: **Mitsubishi Chemical Corporation**
**Tokyo 100-8251 (JP)**

(72) Inventors:
• **OKURANO Takashi**
**Tokyo 100-8251 (JP)**
• **TORII Hisayoshi**
**Tokyo 100-8251 (JP)**
• **KAWAMATA Yuta**
**Tokyo 100-8251 (JP)**
• **SHIOGUCHI Kaoru**
**Tokyo 100-8251 (JP)**
• **TAKAHASHI Eigo**
**Tokyo 100-8251 (JP)**
• **HARA Hideyuki**
**Tokyo 100-8251 (JP)**
• **HARA Kotaro**
**Tokyo 100-8251 (JP)**
• **KOBAYASHI Yoshiaki**
**Tokyo 100-8251 (JP)**

(74) Representative: **Hoffmann Eitle**
**Patent- und Rechtsanwälte PartmbB**
**Arabellastraße 30**
**81925 München (DE)**

(54) **NOVEL SUCROSE FATTY ACID ESTER**

(57)   The present invention relates to a novel sucrose fatty acid ester, and use thereof. Specifically, it relates to a sucrose fatty acid ester, wherein at least a portion of the hydroxyl groups of sucrose are substituted with an aliphatic hydrocarbon groups, wherein an average degree of substitution of the hydroxyl groups is 2.23 to 2.47, and food such as a beverage and a soft candy containing the sucrose fatty acid ester.

**EP 4 631 950 A1**

**Description**

Technical Field

Cross-reference to Related Applications:

This application is based upon and claims the priority of the prior Japanese Patent Application Nos. 2022-194655 (filed on December 6, 2022) and 2023-099241 (filed on June 16, 2023), the entire contents of which are incorporated herein by reference.

The present invention relates to a novel sucrose fatty acid ester. Specifically, the present invention relates to a sucrose fatty acid ester having an average degree of substitution of hydroxyl groups of 2.23 to 2.47, and to use thereof.

Background Art

A sucrose fatty acid ester (hereinafter also referred to as "SE") is a compound in which at least one of eight hydroxyl groups of a sucrose molecular skeleton are esterified with an aliphatic hydrocarbon group (Patent Literature 1). A degree of ester substitution of the SE has a distribution, and is usually indicated by an average degree of substitution (hereinafter also referred to simply as "degree of substitution").

An SE having a low degree of substitution has high hydrophilicity, and is used as an oil-in-water (O/W) emulsion stabilizer or the like for coffee with milk and the like in the food emulsifiers market (Patent Literatures 1 to 5). An SE having a low degree of substitution has, however, a disadvantage of easily making off-flavor typically associated with emulsifiers be felt, and causes a problem in use particularly in sensorily sensitive food such as a beverage. In contrast, an SE having a high degree of substitution has low solubility in water, and is limited in the amount of use in food containing a large amount of water.

Further, owing to the improvement of preservation technology, foods and beverages are distributed and consumed worldwide in more and more cases in these days. According to the standard defined by, for example, The Food and Agriculture Organization (FAO) of the United Nations and World Health Organization (WHO) in "FAO/WHO Joint Expert Committee on Food Additives (JECFA)", it is necessary that a total content of mono-, di-, and triesters is 80% or more in the entire amount, but a sucrose fatty acid ester that satisfies the standard, and is free from off-flavor typically associated with emulsifiers is not known.

A sucrose fatty acid ester is contained as an emulsifier in a gummy or a soft candy (Patent Literatures 6 to 9). There is a known emulsifier composition for soft candies that contains a sucrose fatty acid ester, citric acid monoglyceride, sorbitan fatty acid ester, and diglycerin monostearate so as to suppress exudation of fat and oil, and to impart softness and an effect of preventing sticking to teeth (Patent Literature 10). In addition, it is known that when a sucrose fatty acid ester is further contained in a gummy candy using a sugar alcohol instead of sugar, crystallization of the sugar alcohol can be prevented to provide a gummy candy having preferable texture (Patent Literature 11).

Citation List

Patent Literature

Patent Literature 1: Japanese Patent Laid-Open No. 11-28077
Patent Literature 2: Japanese Patent Laid-Open No. 2004-194653
Patent Literature 3: WO2015/034068
Patent Literature 4: Japanese Patent Laid-Open No. 2019-150021
Patent Literature 5: Japanese Patent Laid-Open No. 2001-064673
Patent Literature 6: Japanese Patent Laid-Open No. 5-227893
Patent Literature 7: Japanese Patent Laid-Open No. 8-280326
Patent Literature 8: Domestic Re-publication of PCT International Application No. 02/009530
Patent Literature 9: Japanese Patent Laid-Open No. 2017-158526
Patent Literature 10: Japanese Patent Laid-Open No. 2007-124937
Patent Literature 11: Japanese Patent Laid-Open No. 2012-105603

Summary of Invention

Technical Problem

A principal object of the present invention is to provide an emulsifier excellent in emulsion stability and excellent in flavor, and food and the like using the emulsifier.

Solution to Problem

The present inventors have found that the above-described problem can be solved by using a sucrose fatty acid ester having an average degree of substitution of hydroxyl groups of 2.23 to 2.47. The present inventors have further found that a problem of stickiness of a soft candy and the like can be solved by using the sucrose fatty acid ester.

Specifically, the present invention provides the following [1] to [13]:

[1] A sucrose fatty acid ester, wherein at least a portion of the hydroxyl groups of sucrose are substituted with aliphatic hydrocarbon groups, wherein an average degree of substitution of the hydroxyl groups is 2.23 to 2.47. The average degree of substitution of the hydroxyl groups is preferably 2.25 to 2.40, more preferably 2.28 to 2.34, and for example, about 2.3.

[2] The sucrose fatty acid ester according to [1], wherein a total ratio of a monoester body and a diester body is 58 to 64%, preferably 59 to 63%, and more preferably 60 to 62%.

[3] The sucrose fatty acid ester according to [1] or [2], wherein 55% or more and 100% or less, preferably 60% or more and 100% or less, and more preferably 65% or more and 100% or less of the aliphatic hydrocarbon groups are stearic acid groups.

[4] An emulsion composition comprising the sucrose fatty acid ester according to any one of [1] to [3].

[5] Food comprising the sucrose fatty acid ester according to any one of [1] to [3].

[6] The emulsion composition according to [4], wherein the emulsion composition is an oil-in-water emulsion composition.

[7] The emulsion composition according to [6], wherein a content of fat and oil is 0.1 to 50% by weight.

[8] The food according to [5], wherein the food is a beverage.

[9] The food according to [8], wherein the beverage is black tea or coffee.

[10] The emulsion composition according to [6] or [7], wherein a content of fat and oil is 30 to 50% by weight.

[11] The food according to [5], wherein the food is whipped cream.

[12] A soft candy comprising the sucrose fatty acid ester according to [1], fat and oil, and a carbohydrate, wherein a content of the fat and oil is 15% by weight or less.

[13] The soft candy according to [12], wherein 35 to 100% by weight of sucrose fatty acid esters contained in the soft candy is the sucrose fatty acid ester according to [1].

[14] The soft candy according to [12] or [13], wherein a content of the sucrose fatty acid ester according to [1] is 0.1 to 10% by weight, preferably 0.3% by weight to 5.0% by weight, more preferably 0.3% by weight to 1.0% by weight, and further preferably 0.3% by weight to 0.7% by weight.

[15] The soft candy according to any one of [12] to [14], wherein the soft candy is a caramel or a chewy candy.

In [12] to [15] described above, in the sucrose fatty acid ester according to [1], a total ratio of a monoester body and a diester body is 58 to 64%, preferably 59 to 63%, and more preferably 60 to 62% in some embodiments; and 55% or more and 100% or less, preferably 60% or more and 100% or less, and more preferably 65% or more and 100% or less of the aliphatic hydrocarbon groups are stearic acid groups in some embodiments.

Advantageous Effects of Invention

A sucrose fatty acid ester of the present invention is excellent in oil-in-water emulsification when dispersed in water, and excellent also in long-term stability. Besides, the sucrose fatty acid ester of the present invention does not have off-flavor typically associated with emulsifiers, and is excellent in flavor. Therefore, the sucrose fatty acid ester of the present invention is suitably used as an emulsifier for food, particularly an emulsifier for sensorily sensitive food such as a beverage and whipped cream. In addition, when the sucrose fatty acid ester of the present invention is used, a soft candy having a necessary shape retention property, and reduced in stickiness (sticky viscosity) to teeth and other candies is provided.

Description of Embodiments

## 1. Sucrose Fatty Acid Ester

A sucrose fatty acid ester is a compound in which at least a portion of eight hydroxyl groups of a sucrose molecular skeleton are esterified with an aliphatic hydrocarbon group.

A sucrose fatty acid ester of the present invention is characterized by an average degree of substitution of hydroxyl groups with fatty acid (referred to as "average degree of ester substitution" or simply as "(average) degree of substitution") of 2.23 to 2.47, preferably 2.25 to 2.40, more preferably 2.28 to 2.34, and for example, about 2.3. When the degree of substitution falls in this range, a sucrose fatty acid ester having high hydrophilicity and excellent in dispersibility in water can be obtained, and as a result, an emulsion composition having high emulsion stability, particularly, an oil-in-water emulsion composition can be provided. Also when it is used in application having a high ratio of fat and oil, and difficult to be dispersed in water, sufficient dispersibility can be obtained. Besides, the sucrose fatty acid ester of the present invention has an advantage that it is excellent in flavor because off-flavor typically associated with emulsifiers is less perceptible even in use in food.

The "average degree of ester substitution" can be confirmed by analyzing the ester composition by gel permeation chromatography, and calculating the average in accordance with the following equation:

Average degree of substitution = [amount of mono-substituent (wt%) $\times$ 1 + amount of di-substituent (wt%) $\times$ 2 + amount of tri-substituent (wt%) $\times$ 3 + amount of tetra-substituent (wt%) $\times$ 4 + amount of penta-substituent (wt%) $\times$ 5] $\times$ 0.01

In the sucrose fatty acid ester of the present invention, a total ratio of a monoester body and a diester body is 58% or more, preferably 58 to 64%, more preferably 59 to 63%, and further preferably 60 to 62%. When the ratio is within this range, the dispersibility in water can be improved, and particularly, even when the content of fat and oil is large, the dispersibility can be retained.

The sucrose fatty acid ester of the present invention has HLB of 3.5 to 4.5, and preferably about 4.

Herein, the term "about" refers to a value varied from a reference value by up to plus or minus 10%, 8%, 6%, 5%, 4%, 3%, 2%, or 1%. Preferably, the term "about" refers to a range within plus or minus 10%, 5%, or 1% from the reference value.

An aliphatic hydrocarbon group contained in the sucrose fatty acid ester of the present invention is preferably a saturated hydrocarbon group for obtaining an oil-in-water emulsion having high emulsion stability. The aliphatic hydrocarbon group is preferably a chain hydrocarbon group, and more preferably a straight-chain hydrocarbon group.

The number of carbon atoms of the aliphatic hydrocarbon group is preferably large from the viewpoints of hydrophilicity, dispersibility in water, and emulsion stability of an oil-in-water emulsifier. On the other hand, the number of carbon atoms of the aliphatic hydrocarbon group is preferably small from the viewpoint that off-flavor typically associated with emulsifiers is less perceptible. Specifically, the number of carbon atoms of the aliphatic hydrocarbon group is preferably 12 or more, and more preferably 16 or more from the viewpoints of emulsion stability and the like, and is preferably 22 or less, and more preferably 18 or less from the viewpoint of flavor.

Specifically, the aliphatic hydrocarbon group contained in the sucrose fatty acid ester of the present invention is preferably a hydrocarbon group derived from palmitic acid, oleic acid, myristic acid, lauric acid, behenic acid, or stearic acid, and more preferably a hydrocarbon group derived from palmitic acid, or stearic acid, and further preferably a hydrocarbon group derived from stearic acid (hereinafter referred to as the "stearic acid group" in some embodiments).

Preferably 55% or more, more preferably 60% or more, and further preferably 65% or more of aliphatic hydrocarbon groups contained in the sucrose fatty acid ester of the present invention are stearic acid groups.

The type of aliphatic hydrocarbon groups contained in the sucrose fatty acid ester of the present invention, and the ratio thereof to the entire amount of aliphatic hydrocarbon groups can be confirmed by high performance liquid chromatography or gas chromatography.

The sucrose fatty acid ester of the present invention can be produced by an esterification reaction between sucrose and fatty acid, an esterification reaction with fatty acid chloride or fatty acid anhydride of sucrose, a transesterification reaction between sucrose and a lower ester compound of fatty acid, a reaction with an enzyme, microwave irradiation of a mixture containing a fatty acid ester or a fatty acid, or the like. A sucrose fatty acid ester having a low degree of substitution can be produced by adjusting the ratio between fatty acid methyl ester and sucrose in any of these production processes.

The sucrose fatty acid ester of the present invention can be used as an emulsion stabilizer (emulsifier), a lubricant, and the like. The sucrose fatty acid ester of the present invention is excellent in oil-in-water emulsification when dispersed in water, and hence can be suitably used particularly as an oil-in-water (O/W) emulsion stabilizer.

The sucrose fatty acid ester of the present invention is excellent in long-term stability as an oil-in-water (O/W) emulsion stabilizer. The long-term stability can be evaluated based on the appearance.

Owing to the above-described properties, the sucrose fatty acid ester of the present invention can be suitably used in applications to food, pharmaceuticals, cosmetics, industrial products, and the like.

In particular, the sucrose fatty acid ester of the present invention has an advantage that it is excellent in flavor because off-flavor typically associated with emulsifiers is less perceptible, and hence can be suitably used as an emulsifier for food, particularly for a beverage.

2. Sucrose Fatty Acid Ester Composition

The sucrose fatty acid ester of the present invention can be used in an appropriate concentration in accordance with the use. For example, when the sucrose fatty acid ester of the present invention is used as an emulsion stabilizer, the amount of the sucrose fatty acid ester in an emulsion composition containing the sucrose fatty acid ester of the present invention (hereinafter referred to as the "emulsion composition of the present invention" in some embodiments) is preferably large from the viewpoint of emulsion stability. On the other hand, in use in food or the like, the amount of the sucrose fatty acid ester of the present invention is preferably small from the viewpoints that it is excellent in dispersibility in the composition, and that taste and texture derived from the sucrose fatty acid ester are difficult to feel. In other words, the amount of the sucrose fatty acid ester in the emulsion composition is preferably 0.0001% by weight or more, more preferably 0.001% by weight or more, and further preferably 0.01% by weight or more from the viewpoint of emulsion stability, and is preferably 1% by weight or less, and more preferably 0.5% by weight or less from the viewpoint of flavor.

When the sucrose fatty acid ester of the present invention is used as an oil-in-water emulsion stabilizer, the amount of the sucrose fatty acid ester in an oil-in-water emulsion composition containing the sucrose fatty acid ester of the present invention (hereinafter referred to as the "oil-in-water emulsion composition of the present invention" in some embodiments) is also preferably 0.001% by weight or more, and more preferably 0.01% by weight or more from the viewpoint of emulsion stability, and is preferably 1% by weight or less, and more preferably 0.5% by weight or less from the viewpoint of flavor for the same reasons as those described above.

Examples of an oil component contained in the oil-in-water emulsion composition containing the sucrose fatty acid ester of the present invention include animal fats such as milk fat and lard, and vegetable fats such as palm oil, coconut oil, rapeseed oil, palm kernel oil, sunflower oil, soybean oil, and almond oil. Among these, vegetable fats are preferred because of low crystallinity, and fat and oil having a low solid fat content (such as soybean oil, rapeseed oil, or rice oil) is more preferred.

A ratio between oil and water contained in the oil-in-water emulsion composition containing the sucrose fatty acid ester of the present invention is not limited as long as the oil-in-water emulsion composition is formed, and the effect of the present invention (being excellent in both the dispersibility and the flavor, particularly the dispersibility) is exhibited particularly in a range where the ratio of fat and oil is high in the oil-in-water emulsion composition. When the content of water is small, "dispersibility in water" is difficult to obtain. From this point of view, the content of fat and oil is preferably 0.1 to 50% by weight, more preferably 20 to 50% by weight, and further preferably 30 to 50% by weight. Examples of such a composition include whipped cream, and coffee whitener.

3. Food Containing Sucrose Fatty Acid Ester of the Invention

As described above, the sucrose fatty acid ester of the present invention can be suitably used in food applications.

The sucrose fatty acid ester of the present invention can be suitably used as an emulsion stabilizer, particularly as an emulsion stabilizer for an oil-in-water emulsion composition. In other words, the sucrose fatty acid ester of the present invention can be suitably used as an emulsion composition.

The sucrose fatty acid ester of the present invention can be particularly suitably used in an oil-in-water emulsion composition.

The food suitable for using the sucrose fatty acid ester of the present invention is not especially limited, and examples thereof include milk beverages such as milk coffee, milk tea, and milk cocoa; and emulsion compositions such as whipped cream, and coffee whitener. Among these, a milk beverage and an emulsion composition are preferred, and milk coffee and whipped cream are particularly preferred.

The content of the sucrose fatty acid ester of the present invention in food is not especially limited, and is preferably large from the viewpoint of emulsion stability. On the other hand, when the sucrose fatty acid ester of the present invention is used in food or the like, the amount thereof is preferably small because it is excellent in dispersibility in a composition, and a taste and texture derived from the sucrose fatty acid ester are difficult to feel. Specifically, the content of the sucrose fatty acid ester of the present invention in food is preferably 0.01% by weight or more, and more preferably 0.1% by weight or more from the viewpoint of emulsion stability, and is preferably 1% by weight or less, and more preferably 0.5% by weight or less from the viewpoint of flavor.

Examples of components contained in the food in addition to the sucrose fatty acid ester of the present invention include an emulsifier except for the sucrose fatty acid ester of the present invention, water, oil, and other food raw materials and food additives. Specific examples of those used in, for example, whipped cream include fresh cream or sodium caseinate, a skim milk powder, and a thickening stabilizer.

The emulsion composition of the present invention can be produced by any of known methods.

The oil-in-water emulsion composition of the present invention is excellent in stability, particularly in long-term stability. The stability of the oil-in-water emulsion composition of the present invention can be evaluated by observing the appearance, and the long-term stability thereof can be evaluated by observing the appearance, and measuring a particle size distribution of oil in the emulsion composition. For example, as change in the appearance such as aggregation, creaming, or separation is smaller, and change in the particle size distribution is smaller, the long-term stability of the oil-in-water emulsion composition of the present invention can be evaluated as better.

In the emulsion composition of the present invention, off-flavor derived from the sucrose fatty acid ester of the present invention is less perceptible. The off-flavor of the emulsion composition of the present invention can be confirmed by sensory evaluation.

Food containing the sucrose fatty acid ester of the present invention as an emulsifier can be produced by using the sucrose fatty acid ester of the present invention as an emulsifier in any known food containing an emulsifier. Specifically, for example, milk coffee containing the sucrose fatty acid ester of the present invention can be produced by a method described in Examples below. Besides, whipped cream containing the sucrose fatty acid ester of the present invention can be produced by a method described in Examples below.

4. Beverage Containing Sucrose Fatty Acid Ester of the Invention

The sucrose fatty acid ester of the present invention is preferably used particularly in application to a beverage.

The content of the sucrose fatty acid ester of the present invention contained in a beverage containing the sucrose fatty acid ester of the present invention (hereinafter simply referred to as the "beverage of the present invention" in some embodiments) is preferably large from the viewpoint of emulsion stability, and specifically, is preferably 300 ppm or more, more preferably 600 ppm or more, and further preferably 800 ppm or more. On the other hand, the content is preferably small from the viewpoint of flavor, and specifically is preferably 3000 ppm or less, more preferably 2000 ppm or less, and further preferably 1800 ppm or less.

The beverage of the present invention may further contain a sucrose fatty acid ester having an average degree of substitution of 1.45 to 2.22. The content of the sucrose fatty acid ester having an average degree of substitution of 1.45 to 2.22 is, however, preferably small from the viewpoint of flavor, and specifically is preferably 400 ppm or less, and more preferably 150 ppm or less. A sucrose fatty acid ester having an average degree of substitution of 1.45 to 2.22 usually has HLB of about 5 to about 12.

The beverage of the present invention may further contain a sucrose fatty acid ester having an average degree of substitution of 1.00 to 1.44. The content of the sucrose fatty acid ester having an average degree of substitution of 1.00 to 1.44 is, however, preferably 100 ppm to 3000 ppm, and more preferably 300 ppm to 2000 ppm from the viewpoint of bacteriostatic property. A sucrose fatty acid ester having an average degree of substitution of 1.00 to 1.44 usually has HLB of about 13 or more (about 13 to about 18).

The beverage of the present invention may contain an ionic emulsifier for further improving the emulsion stability. As the ionic emulsifier, DATEM, succinic acid monoglyceride, citric acid monoglyceride, and SSL are preferred, DATEM and citric acid monoglyceride are more preferred, and DATEM is particularly preferred. The content of the ionic emulsifier contained in the beverage of the present invention is preferably large from the viewpoint of emulsion stability, and specifically is preferably 30 ppm or more, more preferably 75 ppm or more, and further preferably 150 ppm or more. On the other hand, the content is preferably small from the viewpoint of flavor, and specifically is preferably 500 ppm or less, and more preferably 350 ppm or less.

The beverage of the present invention may contain a polyglycerin fatty acid ester having an average degree of polymerization of 2 to 10 for further improving the emulsion stability.

The beverage of the present invention may contain microcrystalline cellulose for suppressing occurrence of precipitation during storage. The content of the microcrystalline cellulose is preferably 200 to 2000 ppm, and more preferably 350 to 1300 ppm.

The beverage of the present invention may contain a stabilizer in addition to the sucrose fatty acid ester described above. Examples of the stabilizer include carrageenan, xanthan gum, guar gum, tara gum, tamarind seed gum, psyllium seed gum, pectin, locust bean gum, and curdlan.

From the viewpoints that flavor is unlikely to be degraded by sterilization, and that preferable flavor can be obtained, the oil contained in the beverage of the present invention is preferably milk fat, coconut oil, palm oil, palm kernel oil, sunflower oil, rapeseed oil, fat and oil obtained by hydrogenating any of these fats or oils, or fat and oil obtained by transesterification of any of these fats or oils, and is more preferably milk fat, coconut oil, palm oil, or palm kernel oil. The content of the fat and oil contained in the beverage of the present invention is preferably 0.1 to 5% by weight, more preferably 0.3 to 4% by weight, and further preferably 0.5 to 2% by weight from the viewpoints of flavor and emulsion stability.

The beverage of the present invention may contain a pH adjuster such as an organic acid or a salt thereof, sodium bicarbonate, or phosphate.

The beverage of the present invention may contain a saccharide, a sugar alcohol, a sweetener, an essence, a flavoring material, a mineral material, a nutritious material, an antioxidant, a preservative, an alcoholic drink, and the like.

Examples of the saccharide include monosaccharides and oligosaccharides such as sugar, granulated sugar, fructose, glucose, maltose, galactose, mannose, fucose, xylose, trehalose, lactose, mannooligosaccharide, and maltooligosaccharide.

Examples of the sugar alcohol include sugar alcohols such as erythritol, xylitol, maltitol, sorbitol, mannitol, and inositol.

Examples of the sweetener include sucralose, aspartame, acesulfame potassium, neotame, and a stevia extract.

Examples of the essence include lemon oil, orange oil, mint oil, coffee flavor, black tea flavor, butter flavoring, cream flavoring, and milk flavoring.

Examples of the flavoring material include carotenoids such as β-carotene, astaxanthin, lycopene, and paprika pigment, pigments such as chlorophyll, and salt.

Examples of the mineral material include salts of calcium, iron, magnesium, and potassium.

Examples of the nutritious material include vitamins, coenzyme Q10, amino acids, peptides, DHA, and EPA.

Examples of the antioxidant include vitamin C, vitamin C sodium salt, vitamin E, a rosemary extract, a tea extract, and a Chinese bayberry extract.

Examples of the preservative include a mustard extract, a shelf life improver such as lysozyme, nicin, sorbic acid, and a salt thereof.

Examples of the alcohol drink include liqueur, vodka, and shochu.

The above-described components and the sucrose fatty acid ester of the present invention are emulsified by mixing appropriately with water or the like, and stirring the resultant. As the emulsification method, any of homogeneous emulsification methods usually employed for food can be employed without limitation. Specifically, any one of a method using a homogenizer, a method using a colloid mill, and a method using a homomixer can be employed.

The homogenous emulsification treatment is usually performed under a heating condition of 40 to 80°C. As homogenous emulsification treatment using a homogenizer, a high-pressure emulsification treatment is also preferably employed. A stable beverage can be obtained when the high-pressure emulsification treatment is performed at a treatment pressure at the time of the high-pressure emulsification in a single-stage method, or at a treatment pressure at the time of at least one stage of high-pressure emulsification in a multi-stage method including two or more stages of usually 5 MPa or more, preferably 10 MPa or more, more preferably 15 MPa or more, further preferably 20 MPa or more, most preferably 25 MPa or more, and usually 200 MPa or less, and preferably 100 MPa or less.

After the homogenous emulsification treatment, sterilization treatment by UHT sterilization, retort sterilization or the like is performed. Retort sterilization is usually performed under conditions of 110 to 140°C, for example, 121°C for 10 to 40 minutes. On the other hand, UHT sterilization employed for plastic bottle beverages is ultra-high temperature sterilization performed at a higher temperature, for example, at a sterilization temperature of 120 to 150°C, and corresponding to a sterilization value (Fo) at 121°C of 10 to 50. The UHT sterilization can be performed by any of known methods including a direct heating method such as a steam injection method in which steam is directly blown into a beverage, and a steam infusion method in which a beverage is sprayed into steam for heating, and an indirect heating method using a surface heat exchanger such as a plate or a tube, and for example, a plate type sterilizer can be used.

Alternatively, the beverage can be produced by compounding the sucrose fatty acid ester of the present invention in an oil-in-water emulsion containing none of a coffee component, a tea component, and a cocoa component, mixing the thus prepared oil-in-water emulsion with a coffee component, a tea component, a cocoa component, or the like, and subjecting the resultant to the homogenization step and sterilization step.

Since off-flavor of the sucrose fatty acid ester of the present invention is difficult to feel, it can be used in coffee, a coffee beverage, a coffee-containing soft drink, a tea beverage, a cocoa beverage, an almond beverage, an oat beverage, coconut milk, rice milk, cashew nut milk, soy milk, a fruit juice beverage, a walnut beverage, barley milk, and macadamia milk. In particular, it is suitably used in a coffee beverage, a tea beverage, an almond beverage, and an oat beverage, and particularly suitably in a coffee beverage.

The beverage of the present invention is suitable to a packaged beverage, and can be applied to, for example, a canned beverage, a plastic bottle beverage, a carton beverage, a bottled beverage, and a plastic container beverage.

5. Coffee or Tea Beverage Containing Sucrose Fatty Acid Ester of the Invention

Coffee according to the present invention encompasses a liquid containing a coffee bean-derived component, such as a solution obtained by extracting ground roasted beans with water or warm water, or a liquid obtained by dissolving, in water or the like, a coffee extract obtained by concentrating the solution, or instant coffee obtained by drying the solution. A coffee bean used as a raw material of a coffee extract used in the present invention is not especially limited, and may be Coffea Arabica or Coffea Canephora. Examples of such a raw bean include those selected from Mexico, Guatemala, blue mountain, crystal mountain, Costa Rica, Columbia, Venezuela, Brazil Santos, Hawaii Cona, Moca, Kenia, Kilimanjaro, Mandheling, and Robusta beans, and mixtures of these.

The coffee extract used in the present invention is preferably extracted with 1 L of water per, for example, 1 to 100 g, and more preferably 20 to 80 g of coffee beans, and the water used in the extraction is preferably at a temperature of 60 to 95°C. Besides, the extraction time for the coffee extract is preferably 30 to 120 minutes. The extraction method of the coffee extract of the present invention is not especially limited, and the extraction can be performed by, for example, a general method of a dripping method, a dipping method, or an espresso method.

A tea beverage (tea-based beverage) according to the present invention is a beverage obtained from tea or a plant other than tea, and encompasses a green tea-based beverage, a black tea beverage, a blended tea beverage, an oolong tea beverage, and a barley tea beverage. The tea beverage of the present invention can be prepared from a tea extract produced by a method usually employed for preparation of a tea extract, or a concentrate or dilute solution thereof. The tea extract used in the present invention can be obtained, for example, by mixing and contacting tea leaves with water (0 to 100°C), or mixing or dissolving a concentrated or purified product of a tea extract, such as a tea extract or a tea powder, with or in water (0 to 100°C). Alternatively, the tea extract obtained by mixing and contacting tea leaves with water may be mixed with the tea extract or tea powder to be used in the tea beverage of the present invention as the tea extract. When tea leaves and water are mixed and contacted with each other, the tea leaves and the resultant tea extract can be separated by a separation method such as centrifugation or filtration. Besides, in the preparation of the tea extract, an optional raw material may be compounded in addition to the tea leaves.

The content of the sucrose fatty acid ester of the present invention contained in the coffee or tea beverage is preferably large from the viewpoint of emulsion stability, and specifically is preferably 300 ppm or more, more preferably 600 ppm or more, and further preferably 800 ppm or more. On the other hand, the content of the sucrose fatty acid ester of the present invention is preferably small from the viewpoint of flavor, and specifically is preferably 3000 ppm or less, more preferably 2000 ppm or less, and further preferably 1800 ppm or less.

The coffee or tea beverage of the present invention may further contain a sucrose fatty acid ester having an average degree of substitution of 1.45 to 2.22. The content of the sucrose fatty acid ester having an average degree of substitution of 1.45 to 2.22 is, however, preferably small from the viewpoint of flavor, and specifically is preferably 400 ppm or less, and more preferably 150 ppm or less. A sucrose fatty acid ester having an average degree of substitution of 1.45 to 2.22 usually has HLB of about 5 to about 12.

The coffee or tea beverage of the present invention may further contain a sucrose fatty acid ester having an average degree of substitution of 1.00 to 1.44. The content of the sucrose fatty acid ester having an average degree of substitution of 1.00 to 1.44 is, however, preferably 100 ppm to 3000 ppm, and more preferably 300 ppm to 2000 ppm from the viewpoint of bacteriostatic property. A sucrose fatty acid ester having an average degree of substitution of 1.00 to 1.44 usually has HLB of about 13 or more.

6. Soft Candy Containing Sucrose Fatty Acid Ester of the Invention

The present invention also provides a soft candy containing the sucrose fatty acid ester of the present invention, fat and oil, and a carbohydrate, and having a content of the fat and oil of 15% by weight or less.

Herein, a soft candy means a candy having softness, and having a water content of 5 to 20% by mass. The principal raw material of the soft candy is a carbohydrate of sugar, mizuame (starch syrup) or the like, and in addition, moisture, fat and oil, gelatin, and the like are contained.

Examples of the type of soft candy include a caramel, a nougat, a marshmallow, and a chewy candy.

The properties of the sucrose fatty acid ester having an average degree of substitution of 2.23 to 2.47 contained in the soft candy of the present invention have been described above in "1. Sucrose Fatty Acid Ester". One of, or two or more of sucrose fatty acid esters having an average degree of substitution of 2.23 to 2.47 may be used.

The soft candy of the present invention may contain, in addition to the sucrose fatty acid ester of the present invention, a sucrose fatty acid ester having an average degree of substitution of 1.45 to 2.22. An example of the sucrose fatty acid ester having an average degree of substitution of 1.45 to 2.22 includes RYOTO sugar ester S-570, manufactured by Mitsubishi Chemical Corporation (average degree of substitution: 2.18, HLB: about 5). A sucrose fatty acid ester having an average degree of substitution of 1.45 to 2.22 has been conventionally used as an emulsifier in a soft candy, and the content thereof in a candy is preferably small as described below because off-flavor typically associated with emulsifiers may be felt. The HLB of a sucrose fatty acid ester having an average degree of substitution of 1.45 to 2.22 is usually about 5 to 12.

The soft candy of the present invention may contain, in addition to the sucrose fatty acid ester of the present invention, a fatty acid ester having an average degree of substitution more than 2.47. An example of the fatty acid ester having an average degree of substitution more than 2.47 includes a commercially available sucrose fatty acid ester having an average degree of substitution of 2.48 to 2.80 (RYOTO sugar ester S-370, manufactured by Mitsubishi Chemical Corporation (average degree of substitution: 2.58, HLB: about 3)).

In the present invention, the content of the sucrose fatty acid ester of the present invention (average degree of substitution: 2.23 to 2.47) contained in the soft candy is preferably large from the viewpoint of emulsion stability, and is preferably small from the viewpoint of flavor. Specifically, the content is preferably 0.1% by weight or more, more preferably 0.3% by weight or more, and further preferably 0.5% by weight or more from the viewpoint of emulsion stability. Specifically, the content is preferably 10% by weight or less, more preferably 5% by weight or less, further preferably 1.0% by weight or less, and most preferably 0.7% by weight or less from the viewpoint of flavor.

In more detail, the content of the sucrose fatty acid ester of the present invention is preferably 0.1 to 10% by weight, more preferably 0.3 to 5% by weight, further preferably 0.3 to 1.0% by weight, and most preferably 0.3 to 0.7% by weight.

The content of the sucrose fatty acid ester having an average degree of substitution of 1.45 to 2.22 contained in the soft candy of the present invention is preferably 0.5% by weight or less, more preferably 0.3% by weight or less, further preferably 0.1% by weight or less, and particularly preferably zero from the viewpoint of flavor.

The content of the fatty acid ester having an average degree of substitution more than 2.47, particularly a sucrose fatty acid ester having an average degree of substitution of 2.48 to 2.80 contained in the soft candy of the present invention is preferably 0.5% by weight or less, more preferably 0.3% by weight or less, further preferably 0.1% by weight or less, and particularly preferably zero from the viewpoint of flavor.

The content of the sucrose fatty acid ester of the present invention (average degree of substitution: 2.23 to 2.47) in the soft candy is preferably 0.1 to 10% by weight, more preferably 0.3 to 5% by weight, further preferably 0.3 to 1.0% by weight, and most preferably 0.3 to 0.7% by weight in entire sucrose fatty acid esters from the viewpoint of reducing stickiness.

The sucrose fatty acid ester used in the present invention may be precedently mixed with oil to be in a state of a homogenized emulsion before being added to the soft candy.

The fat and oil contained in the soft candy of the present invention is not especially limited as long as it is usable in food, particularly in a candy, and is preferably hydrogenated rapeseed oil, hydrogenated palm oil, partially hydrogenated rapeseed oil, partially hydrogenated palm oil, partially hydrogenated coconut oil, or the like from the viewpoint of the melting point.

The content of the fat and oil contained in the soft candy of the present invention is 15% by weight or less based on the total weight of the soft candy. The content of the fat and oil is preferably 0.1 to 10% by weight, more preferably 3 to 8% by weight, and most preferably 5 to 8% by weight from the viewpoints of flavor and emulsion stability.

The saccharide contained in the soft candy of the present invention is not especially limited, and sugar, mizuame, fructose, glucose, granulated sugar, fondant, isomerized sugar, maltose, lactose, xylose, mizuame, honey, maple syrup, coupling sugar, palatinose, isomaltooligosaccharide, fructooligosaccharide, galactooligosaccharide, lactosucrose, xylooligosaccharide, sorbitol, mannitol, maltitol, xylitol, isomalt, erythritol, a hydrogenated starch hydrolysate, and the like can be used.

The content of the saccharide contained in the soft candy of the present invention is preferably 40 to 95% by weight, more preferably 60 to 95% by weight, and most preferably 80 to 90% by weight based on the total weight of the soft candy.

The content of moisture contained in the soft candy of the present invention is preferably 1 to 30% by weight, and most preferably 5 to 20% by weight based on the total weight of the soft candy.

The soft candy of the present invention may contain gelatin for adjusting the hardness of the soft candy, but the content thereof is preferably small from the viewpoint of flavor, and specifically is preferably 10% or less, and more preferably 8% or less.

The soft candy of the present invention may contain citric acid for the purpose of retaining the emulsion stability through adjustment of the pH, and adjusting the flavor. The content of citric acid is 0.1% by weight or more, and preferably 0.2% by weight or more, and 10% by weight or less, and preferably 5% by weight or less.

The soft candy of the present invention may contain a polyglycerin fatty acid ester having an average degree of polymerization of 2 to 10 for the purpose of further improving the emulsion stability.

The soft candy of the present invention may contain an emulsifier in addition to the sucrose fatty acid esters described above. Examples of the emulsifier include a glycerin fatty acid ester, a polyglycerin fatty acid ester, a sorbitan fatty acid ester, an organic acid monoglyceride (e.g., acetic acid monoglyceride, citric acid monoglyceride, succinic acid monoglyceride, or lactic acid monoglyceride), a propylene glycol fatty acid ester, a polyglycerol condensed ricinoleic acid ester, and lecithin.

The soft candy of the present invention may contain a pH adjuster such as an organic acid or a salt thereof, sodium bicarbonate, or phosphate.

**EP 4 631 950 A1**

The soft candy may contain, in addition to the above-described raw materials, a high-intensity sweetener, an acidulant, a stabilizer, a colorant, an essence, dried fruit, nuts, various flavoring raw materials (e.g., a dairy product, tea, coffee, cocoa, a herb, honey, fruit juice, a seasoning, and an extract), vitamins, minerals, dietary fiber, components for making the skin beautiful (e.g., collagen, and hyaluronic acid), glycerin, a polysaccharide thickener, and starch.

The soft candy of the present invention may contain an essence, a flavoring material, a mineral material, a nutritious material, an antioxidant, a preservative, a pigment, an alcoholic drink, and the like.

Examples of the essence include lemon oil, orange oil, mint oil, coffee flavor, black tea flavor, butter flavoring, cream flavoring, and milk flavoring.

Examples of the flavoring material include carotenoids such as β-carotene, astaxanthin, lycopene, and paprika pigment, pigments such as chlorophyll, and salt.

Examples of the mineral material include salts of calcium, iron, magnesium, and potassium.

Examples of the nutritious material include vitamins, coenzyme Q10, amino acids, peptides, DHA, and EPA.

Examples of the antioxidant include vitamin C, vitamin C sodium salt, vitamin E, a rosemary extract, a tea extract, and a Chinese bayberry extract.

Examples of the preservative include a mustard extract, a shelf life improver such as lysozyme, nicin, sorbic acid, and a salt thereof.

The present invention is particularly suitable to a soft candy that does not contain a gelling agent of a naturally derived polysaccharide thickener such as gelatin, pectin, carrageenan, agar, tamarind, gum arabic, hydroxymethyl cellulose, pullulan, locust bean gum, guar gam, gum karaya, gellan gum, curdlan, gum tragacanth, arabinogalactan, furcellaran, carrageenan, psyllium seed gum, tara gum, gum karaya, xanthan gum, curdlan, a soybean polysaccharide, alginic acid, or carboxymethyl cellulose, or that has the content of these of 0.5% or less, and particularly 0.125% or less. When the amount of gelatin is small, the elasticity tends to be low, and therefore, stickiness to teeth becomes a problem in particular.

The soft candy of the present invention has a characteristic of reduced stickiness such as stickiness (sticky viscosity). The stickiness of a soft candy can be measured and evaluated with a rheometer or the like as described in Examples below.

The present application also discloses a method in which the sucrose fatty acid ester of the present invention having an average degree of substitution of 2.23 to 2.47 is compounded in a soft candy to reduce the stickiness thereof. The soft candy contains a saccharide, fat and oil, and the like, and the compound has been described above.

Examples

Now, the present invention will be specifically described by way of examples, and it is noted that the present invention is not limited to these examples.

Example 1: Production of Sucrose Fatty Acid Ester of the Invention

A sucrose fatty acid ester was produced as follows:
1 mole of sucrose was reacted with 0.56 moles of stearic acid methyl ester. The reaction was performed by using, as a solvent, 70% by weight of DMSO with 30% by weight of sucrose and a mixed fatty acid methyl ester of stearic acid and palmitic acid. The reaction was performed with potassium carbonate used as a catalyst in anhydrous state. The reaction was performed at 90°C and 2.7 KPa. The reaction was performed with lower alcohol produced as a by-product distilled off. After completing the reaction, the (potassium carbonate) catalyst was neutralized with lactic acid to obtain a crude sucrose fatty acid ester. The solvent was removed, and the crude sucrose fatty acid ester was purified to obtain a sucrose fatty acid ester (powder) having an average degree of substitution of 2.31. The HLB of this sucrose fatty acid ester was about 4, and stearic acid occupied about 70% of fatty acids contained therein.

Test Example 1: Beverage Containing Sucrose Fatty Acid Ester of the Invention (fat and oil content: 0.68%)

1. Production of Canned Milk Coffee

The sucrose fatty acid ester produced in Example 1, and the following two commercially available sucrose fatty acid esters were used to produce canned milk coffees (Examples 2 to 8, and Comparative Examples 1 and 2) by a method described below.

- Sucrose fatty acid ester of Example 1 (average degree of substitution: 2.31, HLB: about 4)
- Commercially available product, RYOTO sugar ester S-570 manufactured by Mitsubishi Chemical Corporation (average degree of substitution: 2.18, HLB: about 5)

- Commercially available product, RYOTO sugar ester S-1670 manufactured by Mitsubishi Chemical Corporation (average degree of substitution: 1.22, HLB: about 16)

<Organic Acid Monoglyceride>

DATEM: "DATEM517K" manufactured by Danisco
Citric acid monoglyceride: "GRINDSTED CITREM BC/B" manufactured by Danisco

<Milk Component>

Skim milk powder: "Yotsuba Hokkaido Skim Milk" manufactured by Yotsuba Milk Products Co., Ltd.

<Powdered Fat and Oil>

Nestle C40: manufactured by Nestle (fat and oil content: 34.5%)

<Other Components>

Microcrystalline cellulose: "CEOLUS SC-900S" manufactured by Asahi Kasei Chemicals Corporation
Sodium bicarbonate: manufactured by FUJIFILM Wako Pure Chemical Corporation
Granulated sugar: manufactured by Nissin Sugar Manufacturing Co., Ltd.
Instant coffee: Robusta Indonesia, manufactured by Takasago International Corporation

To a coffee liquid obtained by adding a 10-fold amount of hot water to the instant coffee, the sodium bicarbonate previously dissolved in hot water was added to adjust the pH, and then, the respective components were added to and dissolved in the resultant to obtain a composition (% by weight) shown in Table 1, and water was further added thereto. The thus obtained liquid was heated to 65°C, homogenized under a pressure of 20 MPa with a high-pressure homogenizer, and the resultant was filled in a can container. The resultant container was sealed, and subjected to retort sterilization at 121°C for 30 minutes, and thus, a canned milk coffee was produced. The pH of the beverage after the sterilization was 6.8 to 7.0, and a median diameter of oil droplets dispersed in the beverage was 0.15 to 0.20 μm.

[Table 1]

| Instant Coffee | Sodium Bicarbonate | Granulated Sugar | Skim Milk Powder | Powdered Fat and Oil | Stabilizer | Desalted Water |
|---|---|---|---|---|---|---|
| 1.65% | 0.17% | 7.50% | 2.58% | 1.97% | Shown in Table 2 | balance |
| *Stabilizer: Ionic emulsifier (DATEM or citric acid MG), microcrystalline cellulose ||||||| 

The compositions of the sucrose fatty acid esters and the stabilizers contained in the canned milk coffees of Examples 2 to 8, and Comparative Examples 1 and 2 are shown in Table 2.

2. Evaluation of Canned Milk Coffees

The thus obtained canned milk coffees were evaluated as follows, and the results are shown in Table 2.

<Evaluation of Flavor>

The flavor of each milk coffee after the sterilization was evaluated by one trained panelist in accordance with the following criteria:

Good: Astringency of aftertaste is weak, and the flavor is good.
Poor: Astringency of aftertaste is strong, and the flavor is poor.

<Stability after Storage at 60°C for 2 Weeks>

Oil particles: Each canned milk coffee was stored at 60°C for 2 weeks, and then, was allowed to stand still at 4°C overnight. On the next day, a state of creaming caused when the can was opened, and a state of oil particle formation caused when the liquid content was transferred to a cup were visually observed, and evaluated in accordance with the following criteria:

> 1: There are a large amount of oil particles.
> 2: There are a small amount of oil particles.
> 3: Slight creaming is observed in opening the can, but no oil particles are found when the liquid content is transferred to a cup.
> 4: Substantially no creaming is observed in opening the can, no oil particles are found when the liquid content is transferred to a cup, and the liquid content is in a good state.

Precipitation: The bottom of the can after transferring the liquid content to the cup in the evaluation of oil particles was visually observed, and evaluated in accordance with the following criteria:

> 1: There is precipitation.
> 2: There is a small amount of precipitation.
> 3: There is no precipitation, and the can bottom is in a good state.

[Table 2]

| | Sucrose Fatty Acid Ester | | | Ionic Emulsifier | | | | | |
| | Example 1 | S-570 | P-1670 | | | Microcrystalline Cellulose | Flavor | 60°C 2W Stability | 60°C 2W Precipitation |
| Average Degree of Substitution | 2.28 | 2.18 | 1.22 | DATEM | Citric Acid MG | | | | |
| HLB | 4 | 5 | 16 | | | | | | |
| Example 2 | 1000 ppm | - | 700 ppm | - | - | - | good | 2 | 2 |
| Example 3 | 1500 ppm | - | 700 ppm | - | - | - | good | 3 | 2 |
| Example 4 | 1000 ppm | - | 700 ppm | 100 ppm | - | - | good | 4 | 2 |
| Example 5 | 1000 ppm | - | 700 ppm | 200 ppm | - | - | good | 4 | 2 |
| Example 6 | 1000 ppm | - | 700 ppm | 200 ppm | - | 500 ppm | good | 4 | 3 |
| Example 7 | 1000 ppm | - | 700 ppm | 200 ppm | - | 1000 ppm | good | 4 | 3 |
| Example 8 | 1000 ppm | - | 700 ppm | - | 200 ppm | - | good | 4 | 2 |
| Comparative Example 1 | - | 1000 ppm | 700 ppm | - | - | - | poor | 3 | 2 |
| Comparative Example 2 | - | - | 700 ppm | - | - | - | good | 1 | 2 |

3. Results

It was thus proved that the beverages of Examples 2 to 8 containing the sucrose fatty acid ester of the present invention (Example 1) are excellent in the flavor, and mostly good in the emulsion stability. In contrast, the beverage of Comparative Example 1 was inferior in the flavor, and the beverage of Comparative Example 2 was poor in the emulsion stability.

It was proved, based on the results of Examples 4 to 8, that the stability is improved by further containing an ionic emulsifier. It was also proved, based on the results of Examples 6 and 7, that formation of precipitation can be further suppressed by further containing microcrystalline cellulose.

Test Example 2: Whipped Cream Containing Sucrose Fatty Acid Ester of the Invention (fat and oil content: 35.0%)

1. Production of Whipped Cream

The sucrose fatty acid ester produced in Example 1, and the following two commercially available sucrose fatty acid esters were used to produce whipped creams (Example 9, and Comparative Examples 3 and 4) by a method described below.

[Raw Materials Used]

<Sucrose Fatty Acid Ester>

- Sugar ester of Example 1 (average degree of substitution: 2.31, HLB: about 4, stearic acid: 70%)
- Commercially available product, RYOTO sugar ester S-370 manufactured by Mitsubishi Chemical Corporation (average degree of substitution: 2.58, HLB: about 3, stearic acid: 70%)
- Commercially available product, RYOTO sugar ester S-570 manufactured by Mitsubishi Chemical Corporation (average degree of substitution: 2.18, HLB: about 5, stearic acid: 70%)
- Commercially available product, RYOTO sugar ester P-1670 manufactured by Mitsubishi Chemical Corporation (average degree of substitution: 1.22, HLB: about 16, stearic acid: 70%)

<Fat and Oil>

AMF: "Anhydrous Milk Fat" manufactured by Fonterra

<Milk Component>

Fresh cream: "Fresh Cream 45" manufactured by Snow Brand Milk Products Co., Ltd.
Skim milk powder: "Yotsuba Hokkaido Skim Milk" manufactured by Yotsuba Milk Products Co., Ltd.
Sodium caseinate: "Tasua 100" manufactured by Tatua Japan

<Other Emulsifiers>

Soy lecithin: "SLP Paste" manufactured by Tsuji Oil Mills, Co., Ltd.
Glycerin fatty acid ester: "Myverol"

<Other Components>

Guar gum: "RG100" manufactured by Mitsubishi Chemical Corporation
Xanthan gum: "XG800" manufactured by Mitsubishi Chemical Corporation

The amounts (% by weight) of the respective raw materials shown in Table 3 were weighed, the AMF was mixed with the soy lecithin, then, the skim milk powder, the sodium caseinate, the guar gum, the xanthan gum, the glycerin fatty acid ester, and the sucrose fatty acid ester were mixed with water, and to the resultant water mixture was added a mixture of the fresh cream and the oil mixture to obtain a total amount of 100% by weight. The resultant liquid was heated to 70°C, emulsified with a homomixer (7000 rpm), and then homogenized under a pressure of 20 MPa with a high-pressure homogenizer. Thereafter, the resultant was filled in a bottle container, the bottle container was sealed, and cooled with ice water, and thus, a cream mix was prepared. The cream mix after sterilization had a pH of 6.8 to 6.9, a viscosity of 83 to 113, and an initial median diameter of 3.4 to 4.5 μm.

2. Evaluation of Whipped Cream

A whipped cream obtained by whipping the thus obtained cream mix was evaluated as follows, and the results are shown in Tables 3 and 4.

<Evaluation of Flavor>

The flavor of the whipped cream obtained by whipping was evaluated by one trained panelist in accordance with the following criteria:

Good: Astringency of aftertaste is weak, and the flavor is good.
Poor: Astringency of aftertaste is strong, and the flavor is poor.

<Solubility in Water>

Good: The raw materials put into water disperse immediately.
Poor: The raw materials put into water do not disperse immediately but remain undissolved for a while.

<Overrun>

To 400 g of the cream, 30 g of sugar was added, and the resultant was whipped with a Kenmix mixer (130 rpm/19°C). The weight (A) of a prescribed volume of the cream mix before whipping was measured, the weight (B) of the prescribed volume of the whipped mix was measured, and the overrun was obtained in accordance with the following equation:

$$\text{Overrun } [\%] = (\text{before whipping } [g] - \text{after whipping } [g])/\text{after whipping } [g] \times 100$$

100% was set as the minimum level necessary for whipping property.

<Measurement of Hardness>

The whipping operation was performed in the same manner as that for measuring the overrun, and the hardness was measured over time. A prescribed volume of the whipped mix was collected in a 65 ml plastic cup, and the measurement was performed under the following conditions:

Rheometer: RHEO METER CR-500DX (SUN SCIENTIFIC CO., LTD.)
Plunger: 30 mm disc type (No. 14) used
Table speed: 20 mm/min
Maximum load: 2 kg (displayed g)
Unit of load: (g)
Mode: 20
[gf] at a penetration depth of 5 mm measured (600 points)
100% was set as the minimum level necessary for whipping property.

<Observation of Appearance>

It was determined that the whipped cream was in a good state when any of the following (6) to (8) was observed.

(1) Watery (although the mix is creamy and slightly thick)

(2) Watery and having large bubbles

(3) Watery and having an increased number of small/fine bubbles. The mix becomes rather thick (which is confirmed by attaching the mix to the inner wall).

(4) Large bubbles have disappeared, and the mix becomes fine and thick (a track of the whipper starts to appear).

(5) The mix becomes smooth and fine to be creamy. When it is dropped from above, a track is formed.

(6) A soft peak is formed. Slightly rough. Creamy (a peak bent).

(7) A soft peak is formed, and the track clearly remains.

(8) A stiff peak is formed, and the cream becomes yellow. Sufficient for squeezing.

(9) Rather crumbly.

(9) Buttery state.

[Table 3]

|  | Control Example | Example 9 | Comparative Example 3 | Comparative Example 4 |
|---|---|---|---|---|
| 45%Fresh Cream | 51.100% | 51.100% | 51.100% | 51.100% |
| AMF | 12.000% | 12.000% | 12.000% | 12.000% |
| Skim Milk Powder (Yotsuba) | 2.690% | 2.690% | 2.690% | 2.690% |
| MG (Myverol Kerry) | 0.050% | 0.050% | 0.050% | 0.050% |
| Lecithin (SLP paste, Tsuji Oil Mills, Co., Ltd.) | 0.250% | 0.250% | 0.250% | 0.250% |
| Sodium Caseinate (Tatua100) | 0.050% | 0.050% | 0.050% | 0.050% |
| Purified Guar Gum (RG700) | 0.030% | 0.030% | 0.030% | 0.030% |
| Xanthan Gum (XG800) | 0.020% | 0.020% | 0.020% | 0.020% |
| Sucrose Fatty Acid Ester (Example 1) |  | 0.100% |  |  |
| Sucrose Fatty Acid Ester (S-370) |  |  | 0.100% |  |
| Sucrose Fatty Acid Ester (S-570) |  |  |  | 0.100% |
| Flavor | good | good | good | poor |
| Solubility in Water | - | good | poor | good |

[Table 4]

| Control Example | Stirring Time(min) | 2 | 2.5 | 3 | 3.5 | 3.75 | 4 | 4.5 | 5 | 5.25 | 5.5 | 6 | 6.5 | 7 | 7.5 | 8 |
|---|---|---|---|---|---|---|---|---|---|---|---|---|---|---|---|---|
| | Overrun(%) | 85 | | 30 | | 129 | | 140 | 140 | | 108 | 87 | | 62 | | |
| | Hardness(gf) | | | 10 | | 25 | | 63 | 100 | | 147 | 121 | | 120 | | |
| | Appearance | 2 | | 4~5 | | 6.0 | | 8 | 8~9 | | 9 | 9 | | 9 | | |

| Example 9 | Stirring Time(min) | 2 | 2.5 | 3 | 3.5 | 3.75 | 4 | 4.5 | 5 | 5.25 | 5.5 | 6 | 6.5 | 7 | 7.5 | 8 |
|---|---|---|---|---|---|---|---|---|---|---|---|---|---|---|---|---|
| | Overrun(%) | 130 | | 149 | | | 160 | 163 | 163 | 167 | | 141 | 132 | 124 | 117 | |
| | Hardness(gf) | | | 20 | | | 47 | 69 | 102 | 103 | | 105 | 111 | 94 | 91.7 | |
| | Appearance | 2 | | 4 | | | 7 | 7~8 | 7~8 | 9 | | 9 | 9 | 9 | 9 | |

| Comparative Example 3 | Stirring Time(min) | 2 | 2.5 | 3 | 3.5 | 3.75 | 4 | 4.5 | 5 | 5.25 | 5.5 | 6 | 6.5 | 7 | 7.5 | 8 |
|---|---|---|---|---|---|---|---|---|---|---|---|---|---|---|---|---|
| | Overrun(%) | 128 | | 151 | | | 160 | 163 | 162 | 153 | 131 | 126 | 117 | 116 | | |
| | Hardness(gf) | 0 | | 23 | | | 58 | 79 | 117 | 125 | 127 | 113 | 106 | 91.2 | | |
| | Appearance | 2 | | 4 | | | 7 | 7~8 | 8~9 | 9 | 9 | 9 | 9 | 9 | | |

| Comparative Example 4 | Stirring Time(min) | 2 | 2.5 | 3 | 3.5 | 3.75 | 4 | 4.5 | 5 | 5.25 | 5.5 | 6 | 6.5 | 7 | 7.5 | 8 | 8.5 |
|---|---|---|---|---|---|---|---|---|---|---|---|---|---|---|---|---|---|
| | Overrun(%) | 116 | | 147 | | | 165 | 169 | 173 | | 169 | 171 | 163 | 146 | | 134 | 129 |
| | Hardness(gf) | | | 14 | | | 31 | 46 | 58 | | 77 | 91 | 100 | 99 | | 90.5 | 76.3 |
| | Appearance | 2 | | 5 | | | 6~7 | 7 | 7~8 | | 7~8 | 8 | 8~9 | 8~9 | | 8~9 | 8~9 |

3. Results

It was proved that the whipped cream of Example 9 containing the sucrose fatty acid ester of the present invention is excellent in solubility in water and flavor, and has high overrun and high hardness. In contrast, the whipped cream of Comparative Example 3 containing the sucrose fatty acid ester having an average degree of substitution of 2.58 (HLB: 3) was poor in solubility in water, and the whipped cream of Comparative Example 4 containing the sucrose fatty acid ester having an average degree of substitution of 2.18 (HLB: 5) was inferior in flavor, and could not be sufficiently hardened, and hence was insufficient as a whipped cream.

Reference Test Example 1

9999 parts by weight of water was added to 1 part by weight of the sucrose fatty acid ester of Example 1, the resultant was heated to 80°C, and stirred for 10 minutes, and the amount remaining undissolved was observed in accordance with the following criteria:

○: A small amount remains undissolved.

×: A large amount remains undissolved.

Reference Test Example 2

The amount remaining undissolved was observed in the same manner as in Reference Test Example 1 except that 1 part by weight of the sucrose fatty acid ester of Example 1 was changed to 0.57 parts by weight of S-1670 and 0.43 parts by weight of S-170.

[Table 5]

|  | Average Degree of Esterification | Total Ratio of Mono- and Diester to All Esters | Total Ratio of Tri- or Higher Esters to All Esters | Observation of Amount Remaining Undissolved |
|---|---|---|---|---|
| Reference Test Example 1 | 2.3 | 61% | 39% | ○ |
| Reference Test Example 2 | 2.3 | 56% | 44% | × |

Results

It was confirmed that the solubility in water is high when the total ratio of a monoester body and a diester body is 58% or more in all the esters.

Reference Test Example 3

The sucrose fatty acid ester of Example 1 was used to produce a canned milk coffee by a method described below.

- Sucrose fatty acid ester of Example 1 (average degree of substitution: 2.31, HLB: about 4)

- Commercially available product, RYOTO sugar ester P-1670 manufactured by Mitsubishi Chemical Corporation (average degree of substitution: 1.22, HLB: about 16)

<Milk Component>

Skim milk powder: "Yotsuba Hokkaido Skim Milk" manufactured by Yotsuba Milk Products Co., Ltd.

<Powdered Fat and Oil>

Nestle C40: manufactured by Nestle (fat and oil content: 34.5%)

<Other Components>

Sodium bicarbonate: manufactured by FUJIFILM Wako Pure Chemical Corporation

Granulated sugar: manufactured by Nissin Sugar Manufacturing Co., Ltd.

Instant coffee: Robusta Indonesia, manufactured by Takasago International Corporation

To a coffee liquid obtained by adding 16.5 parts by weight of hot water to 1.65 parts by weight of the instant coffee, 0.17 parts of the sodium bicarbonate previously dissolved in hot water was added to adjust the pH, and then, 7.5 parts by weight of the granulated sugar, 2.58 parts by weight of the skimmed milk powder, 1.97 parts of the powdered fat and oil, 0.07 parts by weight of RYOTO sugar ester P-1670, and 0.1 parts by weight of the sucrose fatty acid ester of Example 1 were added to and dissolved in the resultant, and water was further added thereto to make 100 parts by weight.
At this point, all the raw materials were well dispersed, and none remained undissolved.

Reference Test Example 4

The amount remaining undissolved was observed in the same manner as in Reference Test Example 3 except that 0.1 parts by weight of the sucrose fatty acid ester of Example 1 was changed to 0.1 parts by weight of RYOTO sugar ester S-570 manufactured by Mitsubishi Chemical Corporation (average degree of substitution: 2.18, HLB: about 5), and as a result, all the raw materials were well dispersed, and none remained undissolved.

Reference Test Example 5

The amount remaining undissolved was observed in the same manner as in Reference Test Example 3 except that 0.1 parts by weight of the sucrose fatty acid ester of Example 1 was changed to 0.1 parts by weight of RYOTO sugar ester S-370 manufactured by Mitsubishi Chemical Corporation (average degree of substitution: 2.58, HLB: about 3), and as a result, all the raw materials were well dispersed, and none remained undissolved.

Test Example 3: Soft Candy Containing Sucrose Fatty Acid Ester of the Invention

1. Production of Sucrose Fatty Acid Ester

In accordance with Example 1, a sucrose fatty acid ester having an average degree of substitution of 2.31 was produced.

2. Production of Soft Candy

In accordance with Table 6, respective raw materials were weighed. Gelatin was soaked in water for 10 minutes or more to prepare a gelatin solution. Maltose syrup, high fructose corn syrup, granulated sugar, water, palm oil shortening, and an emulsifier were heated to 130° and stirred to prepare a sugar solution (*1). The gelatin solution, fondant, and citric acid (*2) were added to the sugar solution (*1), the resultant was homogeneously stirred, and stirred with an electric whipper for 3 minutes for whipping. The resultant was formed into a size of about 2 cm by about 1 cm with a thickness of 1 cm, and cut into a piece, and each piece was wrapped with parchment paper, and sealed in a moisture-proof bag (stored at room temperature).

<Emulsifier>

- Sucrose fatty acid ester produced in 1 (average degree of substitution: 2.31, HLB: about 4, stearic acid: 70%), hereinafter referred to as "S-470"
- Commercially available product, RYOTO sugar ester S-570 manufactured by Mitsubishi Chemical Corporation (average degree of substitution: 2.18, HLB: about 5, stearic acid: 70%)
- Commercially available product, RYOTO sugar ester S-1670 manufactured by Mitsubishi Chemical Corporation (average degree of substitution: 1.18, HLB: about 16, stearic acid: 70%)

- Commercially available product, lecithin

<Other Raw Materials>

- Maltose syrup: Maltose syrup manufactured by Kajisa Starch Industry Corporation
- High fructose corn syrup: H-100 manufactured by Nihon Shokuhin Kako Co., Ltd.
- Granulated sugar
- Palm oil shortening
- Gelatin: gelatin powder black manufactured by JELEAF INC.
- Fondant
- Citric acid

[Table 6]

| Raw Materials | | Amount(g) | | | | | Ratio(%) |
|---|---|---|---|---|---|---|---|
| | | Comparative Example 1 | Comparative Example 2 | Example | Comparative Example 3 | Comparative Example 4 | (no water) |
| Maltose Syrup | *1 | 10.5 | 10.5 | 10.5 | 10.5 | 10.5 | 2.9% |
| High Fructose Corn Syrup | *1 | 157.5 | 157.5 | 157.5 | 157.5 | 157.5 | 43.5% |
| Granulated Sugar | *1 | 150.0 | 150.0 | 150.0 | 150.0 | 150.0 | 41.4% |
| Water | *1 | 38.0 | 38.0 | 38.0 | 38.0 | 38.0 | - |
| Palm Oil Shortening | *1 | 26.0 | 26.0 | 26.0 | 26.0 | 26.0 | 7.2% |
| Lecithin | *1 | | 1.8 | | | | |
| S-470 | *1 | | | 1.8 | | | |
| S-570 | *1 | | | | 1.8 | | |
| S-1670 | *1 | | | | | 1.8 | 0.5% |
| | | | | | | | |
| Gelatin Powder Black | *2 | 0.5 | 0.5 | 0.5 | 0.5 | 0.5 | 0.1% |
| Water | *2 | 2.5 | 2.5 | 2.5 | 2.5 | 2.5 | - |
| Fondant | *2 | 15.0 | 15.0 | 15.0 | 15.0 | 15.0 | 4.1% |
| Citric Acid | *2 | 1.0 | 1.0 | 1.0 | 1.0 | 1.0 | 0.3% |
| Total | | 401.0 | 402.8 | 402.8 | 402.8 | 402.8 | |

3. Evaluation of Soft Candy

The thus obtained soft candies were evaluated as follows, and the results are shown in Table 7.

<Evaluation of Shape Retention Property>

A state obtained after wrapping the soft candy with parchment paper in the production was evaluated in accordance with the following criteria:

○: The sample retains its shape after shaping, and can be shaped.

×: The sample loses its shape after shaping, and cannot be shaped.

<Sticky Viscosity>

The soft candy of each of Example and Comparative Examples was used as a sample to measure sticky viscosity (stickiness) with a rheometer (texture analyzer (TA. XTplus)). The sample was attached to a resin cylindrical probe having a diameter of 1 cm exclusive to the rheometer, and was fixed on a sample table. The probe was lowered at a rate of 1.3 mm/s for compressing the sample with the probe at a compressive load of 1 kg for 10 seconds. Thereafter, the probe was pulled at a rate of 1.3 mm/s, and a stress peak area in pulling was measured. Specifically, as the stress peak area has a smaller value, the sticky viscosity is lower.

It is noted that a sample that could not be shaped in the evaluation of shape retention property was not confirmed for the sticky viscosity.

[Table 7]

| | | | Example | Comparative Example 3 | Comparative Example 4 | Comparative Example 2 | Comparative Example 1 |
|---|---|---|---|---|---|---|---|
| Composition of Sample | Sucrose Fatty Acid Ester | Type | S-470 | S-570 | S-1670 | Lecithin | Not added |
| | | HLB | 4 | 5 | 16 | - | - |
| | | Average Degree of Ester Substitution | 2.31 | 2.18 | 1.18 | - | - |
| | Concentration | | 0.5% | 0.5% | 0.5% | 0.5% | - |
| Evaluation | Shape Retention Property | | O | ○ | × | ○ | × |
| | Sticky Viscosity Peak Area g/s | | 73 | 145 | - | 149 | - |
| Shape retention property: ×: cannot be shaped, or shape of sample lost even if shaped. ○: can be shaped | | | | | | | |

Results

It was confirmed that the soft candy of Example containing the sucrose fatty acid ester having an average degree of substitution of 2.31 had shape retention property, and had low sticky viscosity. In contrast, the soft candies of Comparative Examples containing S-570 or lecithin had high sticky viscosity, and the soft candy of Comparative Example containing S-1670 was insufficient in the shape retention property.

Industrial Applicability

A sucrose fatty acid ester of the present invention can provide an oil-in-water composition excellent in solubility in water and flavor, and having high long-term stability. Accordingly, the sucrose fatty acid ester of the present invention is useful as an emulsifier for food, particularly as an emulsifier for sensorily sensitive food such as a beverage and whipped cream. Besides, when the sucrose fatty acid ester of the present invention is used, a soft candy having shape retention property and having reduced stickiness is provided.

The prior art documents cited herein are all incorporated herein by reference.

**Claims**

1. A sucrose fatty acid ester, wherein at least a portion of the hydroxyl groups of sucrose are substituted with aliphatic hydrocarbon groups, wherein an average degree of substitution of the hydroxyl groups is 2.23 to 2.47.

2. The sucrose fatty acid ester according to claim 1, wherein a total ratio of a monoester body and a diester body is 58% or more.

3. The sucrose fatty acid ester according to claim 1, wherein 55% or more and 100% or less of the aliphatic hydrocarbon groups are stearic acid groups.

4. An emulsion composition comprising the sucrose fatty acid ester according to any one of claims 1 to 3.

5. Food comprising the sucrose fatty acid ester according to any one of claims 1 to 3.

6. The emulsion composition according to claim 4, wherein the emulsion composition is an oil-in-water emulsion composition.

7. The emulsion composition according to claim 6, wherein a content of fat and oil is 0.1 to 50% by weight.

8. The food according to claim 5, wherein the food is a beverage.

9. The food according to claim 8, wherein the beverage is black tea or coffee.

10. The emulsion composition according to claim 6, wherein a content of fat and oil is 30 to 50% by weight.

11. The food according to claim 5, wherein the food is whipped cream.

12. A soft candy comprising the sucrose fatty acid ester according to claim 1, fat and oil, and a carbohydrate, wherein a content of the fat and oil is 15% by weight or less.

13. The soft candy according to claim 12, wherein 35 to 100% by weight of sucrose fatty acid esters contained in the soft candy is the sucrose fatty acid ester according to claim 1.

14. The soft candy according to claim 12, wherein a content of the sucrose fatty acid ester according to claim 1 is 0.1 to 10% by weight.

15. The soft candy according to any one of claims 12 to 14, wherein the soft candy is a caramel or a chewy candy.

## INTERNATIONAL SEARCH REPORT

| International application No. |
|---|
| **PCT/JP2023/043468** |

### A. CLASSIFICATION OF SUBJECT MATTER

*C07H 13/06*(2006.01)i; *A23C 13/14*(2006.01)i; *A23D 7/00*(2006.01)i; *A23F 5/00*(2006.01)i; *A23G 3/34*(2006.01)i; *A23L 9/20*(2016.01)i

FI:  C07H13/06; A23C13/14; A23D7/00 508; A23F5/00; A23L9/20; A23G3/34 101

According to International Patent Classification (IPC) or to both national classification and IPC

### B. FIELDS SEARCHED

Minimum documentation searched (classification system followed by classification symbols)

C07H13/06; A23C13/14; A23D7/00; A23F5/00; A23G3/34; A23L9/20

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | JP 2009-214079 A (DKS CO. LTD.) 24 September 2009 (2009-09-24) claims, paragraphs [0003], [0007]-[0014], [0035], table 1, examples 1-3, comparative example 4, etc. | 1-7 |
| Y | | 8-15 |
| X | JP 2009-215262 A (DKS CO. LTD.) 24 September 2009 (2009-09-24) claims, paragraphs [0003], [0007]-[0012], [0018], [0035], table 1, comparative examples 4, 5, 8 | 1-7 |
| Y | | 8-15 |
| X | JP 2006-169237 A (MITSUBISHI CHEMICAL CORPORATION) 29 June 2006 (2006-06-29) claims, paragraph [0003], comparative example 1, table 7 | 1-9 |
| Y | | 10-15 |
| Y | JP 10-70956 A (MITSUBISHI CHEMICAL CORPORATION) 17 March 1998 (1998-03-17) claims, paragraph [0008], examples | 1-15 |

☑ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **01 February 2024** | **20 February 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT

| International application No. |
|---|
| **PCT/JP2023/043468** |

**C. DOCUMENTS CONSIDERED TO BE RELEVANT**

| Category* | Citation of document, with indication, where appropriate, of the relevant passages | Relevant to claim No. |
|---|---|---|
| Y | JP 2019-150021 A (TAIYO KAGAKU CO., LTD.) 12 September 2019 (2019-09-12)<br>claims, paragraph [0001], examples | 1-15 |
| Y | JP 2014-113123 A (MIYOSHI OIL & FAT CO., LTD.) 26 June 2014 (2014-06-26)<br>claims, paragraphs [0002]-[0005], examples | 1-15 |
| Y | JP 2015-57948 A (SAN-EI GEN F.F.I., INC.) 30 March 2015 (2015-03-30)<br>claims, paragraphs [0001]-[0006], examples | 1-15 |
| Y | WO 2011/129251 A1 (FUJI OIL CO., LTD.) 20 October 2011 (2011-10-20)<br>claims, paragraph [0005], examples | 1-15 |
| Y | JP 2012-65577 A (MITSUBISHI-KAGAKU FOODS CORP) 05 April 2012 (2012-04-05)<br>claims, paragraphs [0003]-[0006], examples | 1-15 |
| Y | JP 2016-67327 A (MEIJI CHIYUUINGAMU KK) 09 May 2016 (2016-05-09)<br>claims, paragraphs [0002], [0006], examples | 1-15 |

Form PCT/ISA/210 (second sheet) (July 2022)

**INTERNATIONAL SEARCH REPORT**
Information on patent family members

International application No.

**PCT/JP2023/043468**

| Patent document cited in search report | | | Publication date (day/month/year) | Patent family member(s) | Publication date (day/month/year) |
|---|---|---|---|---|---|
| JP | 2009-214079 | A | 24 September 2009 | (Family: none) | |
| JP | 2009-215262 | A | 24 September 2009 | (Family: none) | |
| JP | 2006-169237 | A | 29 June 2006 | EP 1813622 A1<br>claims, paragraph [0003],<br>comparative example 1, table 7<br>KR 10-2007-0084228 A<br>CN 101056882 A | |
| JP | 10-70956 | A | 17 March 1998 | (Family: none) | |
| JP | 2019-150021 | A | 12 September 2019 | (Family: none) | |
| JP | 2014-113123 | A | 26 June 2014 | (Family: none) | |
| JP | 2015-57948 | A | 30 March 2015 | (Family: none) | |
| WO | 2011/129251 | A1 | 20 October 2011 | (Family: none) | |
| JP | 2012-65577 | A | 05 April 2012 | (Family: none) | |
| JP | 2016-67327 | A | 09 May 2016 | (Family: none) | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022194655 A
- JP 2023099241 A
- JP 11028077 A
- JP 2004194653 A
- WO 2015034068 A
- JP 2019150021 A
- JP 2001064673 A
- JP 5227893 A
- JP 8280326 A
- WO 02009530 A
- JP 2017158526 A
- JP 2007124937 A
- JP 2012105603 A